(19) Europäisches Patentamt

European Patent Office

Office européen des brevets

(11) Veröffentlichungsnummer: **0 472 772 A1**

## (12) EUROPÄISCHE PATENTANMELDUNG

(21) Anmeldenummer: **90116742.9**

(22) Anmeldetag: **31.08.90**

(51) Int. Cl.5: **A61K 9/50**, A61K 35/18

(43) Veröffentlichungstag der Anmeldung:
**04.03.92 Patentblatt 92/10**

(84) Benannte Vertragsstaaten:
**AT CH DE ES FR GB IT LI NL**

(71) Anmelder: **Krüss GmbH**
**Borsteler Chaussee 85 - 99a**
**W-2000 Hamburg 61(DE)**

(72) Erfinder: **Weser, Cornelius**

Merkelweg 8
**W-2000 Hamburg 61(DE)**
Erfinder: **Schütt, Klaus-Hermann, Dr. med.**
**Schillerstrasse 34**
**W-6501 Budenheim(DE)**

(74) Vertreter: **UEXKÜLL & STOLBERG**
**Patentanwälte**
**Beselerstrasse 4**
**W-2000 Hamburg 52(DE)**

(54) **Verfahren zur Herstellung vitaler Wirkstoff-beladener Humanerythrozyten.**

(57) Es wird ein Verfahren zum Herstellen Wirkstoff-beladener Humanerythrozyten durch Elektroporation, Versiegeln und Rejuvenilisieren aus Humanblut isolierter Erythrozyten beschrieben, welches vitale Erythrozyten mit gegenüber Normalerythrozyten unveränderten Stoffwechseleigenschaften, Oberflächenantigenen und Partikelgrößen liefert. Das Verfahren ist insbesondere zur Herstellung Inositolhexaphosphat-beladener Erythrozyten mit erhöhter Sauerstoff-Abgabebereitschaft geeignet.

Abb. 5

Sauerstoffbindungskurven raffinose- und IHP-modifizierter Erythrozyten im Vergleich zur normalen Sauerstoff-bindungskurve

Die Erfindung betrifft ein Verfahren zum Herstellen vitaler Wirkstoff-beladener Humanerythrozyten, bei welchem die Erythrozytenmembranen durch elektrische Impulse mit Poren versehen und die porösen Erythrozyten mit dem jeweils gewünschten Wirkstoff beladen und anschließend versiegelt und rejuvenilisiert werden. Vorzugsweise werden die Erythrozyten mit einem allosterischen Hämoglobinaktivator wie Inositolhexaphosphat beladen, um auf diese Weise ein klinisch verwendbares Erythrozytenpräparat mit erhöhter Fähigkeit zur Sauerstoffabgabe zu schaffen.

Die Verwendung von Erythrozyten als Carrier für Wirkstoffe und insbesondere die Beladung von Erythrozyten mit allosterischen Hämoglobinaktivatoren zur Steigerung der Sauerstoff-Abgabebereitschaft, ist in der Literatur wiederholt diskutiert worden. Von Gersonde (vgl. K Gersonde und C. Nicolau "Improvement of the Red Blood Cell $O_2$ Release by Lipid Vesicle-mediated Incorporation of Inositol Hexaphosphate", Blut 39, Seiten 1 bis 7 (1979)) ist erstmals ein Verfahren zum Beladen menschlicher Erythrozyten mit Inositolhexaphosphat (IHP) beschrieben worden. Der Wirkstoff wurde in Lipidvesikel eingeschlossen und durch Phagozytose in menschliche Erythrozyten eingeschleust. Da reife Erythrozyten jedoch nur noch eine geringe Phagozytosefähigkeit aufweisen, kann auf diese Weise nur eine niedrige Beladungsfrequenz mit schlecht reproduzierbaren Ergebnissen erreicht werden.

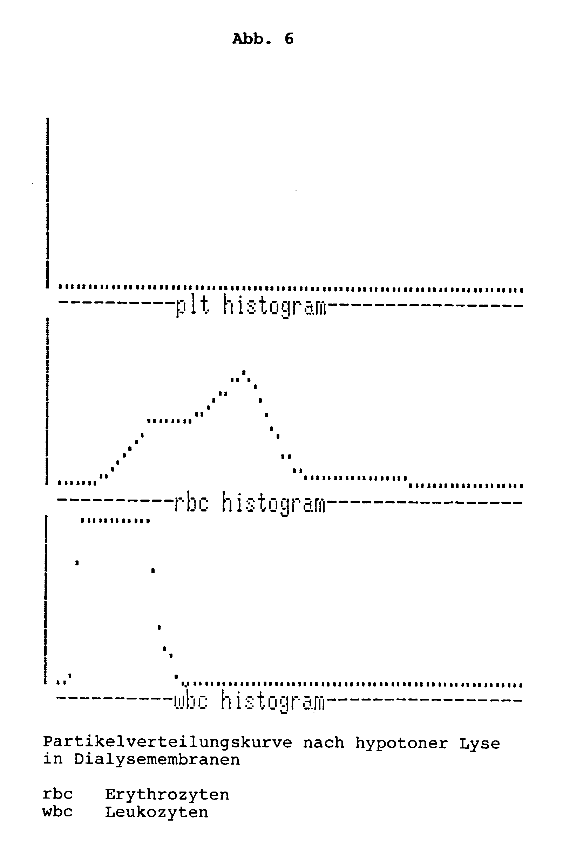

In erheblichem Umfang Wirkstoff-beladene Erythrozyten wurden erstmals von Ropars (vgl. EP 101 341 sowie C. Ropars, M. Chassaigene, M.C. Villereal, G. Avenard, C. Hurel und C. Nicolau "Resealed Red Blood Cells as a New Blood Transfusion Product", Biblthca haemat. 51, Seiten 82 bis 91 (1985)) hergestellt. Das von Ropars verwendete Verfahren beruht auf einer im wesentlichen vollständigen Lysis der Zellmembran mit nachfolgender Reorganisation und damit einhergehendem Einschluß des in dem Gemisch vorhandenem Wirkstoffes nach dem Zufallsprinzip. Bei der Reorganisation gehen jedoch zwangsläufig Zell-Inhaltsstoffe verloren. Ein erheblicher Teil der erhaltenen Zellen ist demgemäß gegenüber den Ausgangszellen im Durchmesser verändert (vgl. Abbildung 6) und weist eine geringere Überlebensdauer auf. Ferner haben erste klinische Studien (vgl. A. Zanella, F. Rosse, L. Sabbioneda, A. Brovelli, und G. Sirchia, "Desferrioxamine Entrapment in Standard Red Cell Concentrates", Transfusion, Band 27, Nummer 6, Seite 528 (1987)) gezeigt, daß die nach dem Ropars-Verfahren hergestellten Erythrozyten einen Methämoglobinanteil von etwa 50% aufweisen. Dies bedeutet, daß die Hälfte des Hämoglobins dieser Zellen nicht für den Sauerstofftransport zur Verfügung steht. Der angestrebte Erfolg, nämlich ein Transfusionspräparat mit erhöhter Sauerstoffkapazität und Abgabebereitschaft, ist damit zunichte gemacht.

Es ist demgemäß Aufgabe der Erfindung, ein Verfahren zur Herstellung von Wirkstoff-beladenen Erythrozyten zu schaffen, bei welchem die Nachteile der oben beschriebenen Verfahren, insbesondere der Verlust von Hämoglobin und die Bildung von Methämoglobin, vermieden werden und welches Erythrozyten mit normaler Größenverteilung und Vitalität und mit normalen immunologischen Eigenschaften der Membranoberfläche liefert.

Zur Lösung der Aufgabe wird das Verfahren nach Anspruch 1 vorgeschlagen; die Ansprüche 2 bis 7 betreffen bevorzugte Ausführungsformen.

Die Beladung von Erythrozyten über elektrisch induzierte Poren als Alternative zur vollständigen hypotonen Lyse ist bereits von Kinosita und Tsong (vgl. Nature 268, 438 bis 441 (1977), Nature 272, 258 bis 260 (1978) und Bioblthca Hemat. 51, 198 bis 114 (1985)) vorgeschlagen worden. Die Autoren schleusten Sucrose mittels elektrischer Pulse in Erythrozyten ein; die anschließend versiegelten Erythrozyten konnten 3 Tage lang in vitro gelagert werden. Nach dem beschriebenen Verfahren wurde die Porenbildung in isotonem Inkubationsmedium (bezogen auf Serum) induziert, wobei das Medium etwa 0,9% Natriumchlorid enthielt und das Verhältnis von $Na^+$:$Ka^+$-Ionen etwa 19:1 betrug. Die Versiegelung der beladenen Erythrozyten wurde von den Autoren durch Erwärmen des Mediums auf 37°C in Gegenwart von Stachyose als osmotischen Balancer durchgeführt. Nur 60% der auf diese Weise versiegelten Erythrozyten überlebten jedoch eine 3-tägige in vitro Inkubation bei 37°C in einem simulierten physiologischen Medium.

Das Verfahren wurde von den Autoren selbst nicht weiterverfolgt und in einer Gegenüberstellung der bis zum Jahre 1985 bekannten Verfahren zur Herstellung von Carrier-Erythrozyten (Bioblthca Hemat. 51, 15 bis 24 (1985)) wird die isoosmotische Elektrodenporation als unbefriedigend bezeichnet, da sie zu starkem Hämoglobinverlust und geringer Wirkstoffaufnahme führe (vgl. Seite 17/18).

Ausgehend von diesen Untersuchungen hat es sich erfindungsgemäß überraschenderweise gezeigt, daß Wirkstoff-beladene Humanerythrozyten mit ausgezeichneter Vitalität und normalen immunologischen Membraneigenschaften hergestellt werden können, wenn man Elektroporation, Beladung und Versiegelung jeweils in den erfindungsgemäßen Medien durchführt. Die nachfolgende Rejuvenilisierung kann nach bekannten Verfahren erfolgen.

Für die Beladung mit Wirkstoffen geeignete Erythrozyten können nach bekannten Verfahren beispielsweise durch Filtration aus Blutkonserven gewonnen werden. Vorzugsweise werden die Erythrozyten nach der Filtration in physiologischer Kochsalzlösung gewaschen.

Gesunde Erythrozyten weisen einen mittleren Durchmesser von 6,4 $\mu$m ± 1,9 $\mu$m sowie eine symmetrische Größenverteilungskurve auf. Diese kann durch Streulichtsignale eines Laser-Hämatologiezählers (beispielsweise ELT 15 Ortho Instruments) ermittelt und sichtbar gemacht werden.

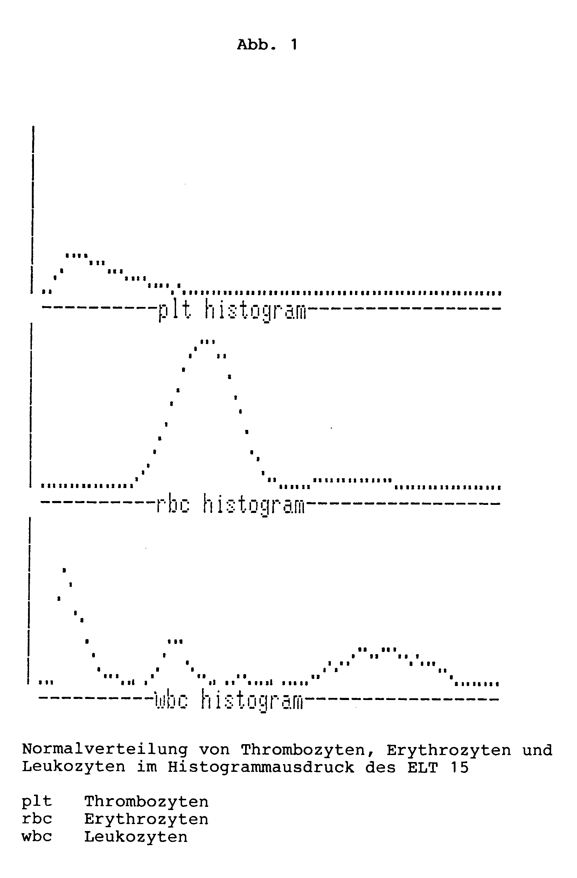

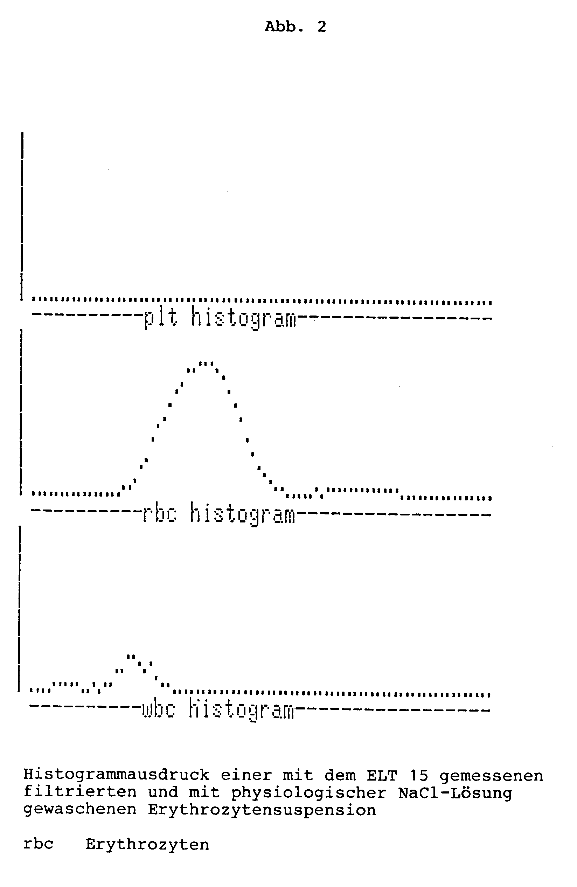

Das Gerät ermöglicht die Aufzeichnung der Histogramme von Thrombozyten (PLT), Erythrozyten (RBC) und Leukozyten (WBC). Die Normalverteilungen von Vollblut sowie einer filtrierten und mit physiologischer NaCl-Lösung gewaschenen Erythrozytensuspension sind in den Abbildungen 1 und 2 wiedergegeben. Letztere dient als Standard für die Beurteilung der nach dem erfindungsgemäßen Verfahren hergestellten Erythrozyten.

Für die erfindungsgemäße Elektroporation werden die Erythrozyten von der physiologischen Kochsalzlösung beispielsweise durch Zentrifugieren abgetrennt und in einer Konzentration von beispielsweise 40 bis 80 Vol.%, vorzugsweise 50 Vol.% in dem erfindungsgemäßen Elektroporations-Medium suspendiert. Entgegen den im Stand der Technik in dieser Stufe verwendeten, bezogen auf die Serumosmolarität isotonen Medien mit hohem Natriumgehalt wird erfindungsgemäß ein leicht hypertones Medium eingesetzt, welches ein $Na^+$-$K^+$-Verhältnis von 3:1 bis 1:3 aufweist. Die Osmolarität des erfindungsgemäßen Mediums beträgt 290 bis 500 mOsmol/l, vorzugsweise 400 bis 450 mOsmol/l und die Leitfähigkeit etwa 5 bis 8 mS.

Als besonders geeignetes Medium hat sich in dieser Stufe Sterofundin[R] B erwiesen, welches die nachfolgend angegebene Zusammensetzung aufweist:

etwa 54 mMol/l $Na^+$,

etwa 24 mMol/l $K^+$,

etwa 2,5 mMol/l $Mg^{++}$,

etwa 51 mMol/l $Cl^-$,

etwa 25 mMol/l $Lactat^-$ und

etwa 7,5 mMol/l $H_2PO^-_4$

etwa 50 g/l Glucose.

Die Temperatur des Mediums kann 0 bis 8°C, vorzugsweise 0 bis 4°C und in besonders bevorzugter Weise 1 bis 3°C betragen. Die angewandte Pulsenergie hängt von der gewünschten Porengröße und damit von der Größe des für die Beladung vorgesehenen Wirkstoffmoleküls ab.

Erfindungsgemäß kommen als Wirkstoffe solche pharmazeutisch wirksamen Substanzen in Betracht, welche die intakte Erythrozytenmembran nicht permeieren können. Gemäß einer besonders bevorzugten Ausführungsform der Erfindung werden die Erythrozyten mit einem allosterischen Hämoglobinaktivator und insbesondere mit Inositolhexaphosphat beladen. Für diesen Fall hat sich bei der Elektroporation eine Feldstärke von 1 bis 6 kV/cm, vorzugsweise von 3 bis 4 kV/cm bei einer Pulsdauer von 20 bis 100 $\mu$s, vorzugsweise von 60 bis 90 $\mu$s und insbesondere von 80 $\mu$s als besonders geeignet erwiesen.

Nach Induktion der Poren werden die Erythrozyten mit der Wirkstofflösung in Kontakt gebracht, wobei der Wirkstoff vorzugsweise dem Elektroporations-Medium direkt zugesetzt wird. Die Erythrozyten können zwar auch zunächst von dem Porations-Medium abgetrennt und anschließend in frischem, den Wirkstoff enthaltenden Medium suspendiert werden. In diesem Fall muß jedoch Sorge dafür getragen werden, daß die in äußerst labilem Zustand befindlichen Membranen nicht irritiert werden.

Es ist ausschlaggebend, die Temperatur des Mediums in dieser Verfahrensstufe bei 0 bis 8°C und vorzugsweise bei 4°C zu halten, da die Poren nur bei niedrigen Temperaturen geöffnet bleiben. Die Zusammensetzung des Mediums während der Beladung sollte bezüglich des Verhältnisses von $Na^+$:$K^+$-Ionen etwa dem Porationsmedium entsprechen. Insbesondere darf die Konzentration der $Na^+$-Ionen die angegebene Obergrenze nicht übersteigen. Wird demgemäß ein Wirkstoff verwendet, der $Na^+$-Ionen in das Medium entlassen kann, so muß dieser zunächst in die Kaliumform überführt werden. Dies gilt insbesondere für das erfindungsgemäß bevorzugte Inositolhexaphosphat. Dieses ist als stark basisches Natriumsalz ($Na_{12}IHP$) im Handel erhältlich. Zur Herstellung einer natriumarmen Lösung des Wirkstoffes kann dieser in Wasser gelöst und solange mit saurem Ionenaustauschharz (beispielsweise Dowfax[R] 50W-X16) versetzt werden, bis der pH-Wert auf etwa 1 bis 2 gefallen ist. Nach Abtrennen des Ionenaustauschers kann das klare Filtrat mit Kaliumhydroxid neutralisiert werden.

Gemäß einer bevorzugten Ausführungsform werden die permeablen Erythrozyten zur Beladung 15 bis 30 Minuten lang in einem Medium inkubiert, welches aus 2 Teilen des erfindungsgemäßen Porationsmediums wie beispielsweise Sterofundin® und 1 Teil der oben beschriebenen Lösung von Inositolhexaphosphat bei einer IHP-Konzentration von etwa 5% bezogen auf dass Gesamtmedium besteht.

Im Anschluß an die Inkubation werden die Zellen von dem Medium beispielsweise durch Zentrifugieren abgetrennt, und anschließend bei 36 bis 41°C, vorzugsweise bei 37°C in dem erfindungsgemäßen Versiegelungs-Medium versiegelt. Die Zusammensetzung dieses Mediums trägt wesentlich zu dem erfindungsgemäß erzielten Erfolg bei. Im Gegensatz zu den im Stand der Technik bisher für die Versiegelung verwendeten Medien auf Basis physiologischer Kochsalzlösung, sind in dem erfindungsgemäß verwendeten Medium keine oder nur eine geringe Menge Natriumionen vorhanden, während es eine hohe Kaliumionen-Konzentration aufweist.

Überraschenderweise hat es sich nämlich erfindungsgemäß gezeigt, daß die Versiegelung der Erythrozyten ohne Hämoglobinverlust und ohne Verlust sonstiger Zellinhaltsstoffe erfolgt, wenn man ein Medium verwendet, welches 80 bis 100 mVal/l $K^+$-Ionen und 0 bis 20 mVal $Na^+$-Ionen enthält. Zusätzlich muß in dem Medium ein Schutzkolloid vorhanden sein. Als solches hat sich Humanalbumin in einer Menge von 25 bis 35 Gew.%, vorzugsweise 30 Gew.% bezogen auf das Medium besonders bewährt. Anstelle von Humanalbumin können auch Humanalbumin-Ersatzstoffe mit den gleichen osmotischen Eigenschaften eingesetzt werden.

Vorzugsweise enthält das erfindungsgemäße Versiegelungsmedium ferner etwa 1 bis 5 mVal und vorzugsweise 3 mVal/l $Mg^+$-Ionen sowie gegebenenfalls 5 bis 20 mMol/l L-Ascorbinsäure und weitere übliche Bestandteile, wobei die Anionen in dem Medium vorzugsweise durch 1,5 bis 2,5 mMol/l anorganische Phosphationen gebildet und der verbleibende Teil durch Chlorionen gestellt werden kann.

Gemäß einer besonders bevorzugten Ausführungsform der Erfindung wird ein Versiegelungsmedium der folgenden Zusammensetzung verwendet:

0,1 Mol Kaliumchlorid

0,012 Mol Na-di-hydrogenphosphat

0,01 Mol L-Ascorbinsäure

0,003 Mol Magnesiumchlorid

30 Gew.% bezogen auf das Endvolumen der Lösung entsalzenes Humanalbumin.

Da kommerziell erhältliches Albumin große NaCl-Mengen enthält, muß es zunächst beispielsweise in einer Filterkammer mit entsprechenden Filtermembranen (beispielsweise Omegacell Filterkammer, Filtron, Membran 30 K-Dalton) entsalzen werden.

Zum Versiegeln werden die Erythrozyten etwa 60 Minuten lang bei 37 - 41 °C in dem erfindungsgemäßen Medium inkubiert. Vorzugsweise wird der Ansatz anschließend 12 bis 24 Stunden lang kühl gehalten.

Zur Rekonstitution des Stoffwechsels der beladenen Erythrozyten ist eine Rejuvenilisierung erforderlich. In dieser Stufe können die in der Transfusionsmedizin bekannten Verfahren zur Rekonstitution des ATP- und 2,3 DPG-Spiegels gelagerter Erythrozyten dienen. Durch die Rejuvenilisierung erhalten die gelagerten Erythrozyten ihre normale Sauerstofftransportfähigkeit und Verformbarkeit zurück.

Die Rejuvenilisierung kann sowohl bei erhöhter Temperatur, wie beispielsweise 37 °C, über einen Zeitraum von 30 bis 60 Minuten als auch über mehrere Stunden bei niedriger Temperatur, wie beispielsweise 4 °C, erfolgen. Als besonders vorteilhaft hat sich die Rejuvenilisierung bei etwa 4 bis 8 °C unter Zusatz eines Medius erwiesen, welches die folgende Zusammensetzung aufweist:

| | |
|---|---|
| Glucose | 3,44 g/l |
| Inosin | 26,8 g/l |
| Na-Pyruvat | 13,75 g/l |
| Adenin | 0,675 g/l |
| Di-Na-hydrogenphosphat $7H_2O$ | 55,0 g/l |
| Benzylalkohol | 10,0 g/l. |

Dieses Medium kann dem Versiegelungsmedium beispielsweise im Verhältnis von etwa 0,3 zu 1 zugegeben werden.

Selbstverständlich muß der pH-Wert sämtlicher in dem erfindungsgemäßen Verfahren verwendeter Medien im physiologischen Bereich, d. h. im Bereich von etwa 6 bis 7,5 liegen.

Die nach dem erfindungsgemäßen Verfahren hergestellten Erythrozyten weisen eine Partikelverteilungskurve auf, welche vollständig mit derjenigen gesunder, unbehandelter Erythrozyten übereinstimmt (vgl. Abbildungen 3 und 4). Im Gegensatz dazu werden nach dem von Ropars beschriebenen Verfahren Partikel mit einer sehr heterogenen Verteilungskurve gefunden, die zum einen deutliche Verschiebungen in den kleineren Partikelbereich und zum anderen eine Partikelfraktion aufweist, die bereits im Lymphozytenkanal erscheint, wobei allerdings die hier gezählten Partikel etwas kleiner sind als Lymphozyten (vgl. Abbildung 6).

Ferner konnte gezeigt werden, daß der ATP-Gehalt der nach dem erfindungsgemäßen Verfahren beladenen Erythrozyten mit demjenigen der Ausgangserythrozyten identisch ist. Nach der Rejuvenilisierung wurde erfindungsgemäß eine ATP-Neusynthese von fast 3 $\mu$Mol ATP/g Hämoglobin gefunden. Dieses Ergebnis läßt den Schluß auf einen intakten Stoffwechsel der rejuvenilisierten Erythrozyten zu.

Schließlich zeigte auch die Überprüfung der Oberflächenantigenstruktur der erfindungsgemäß behandelten Erythrozyten eine vollständige Übereinstimmung mit den Ausgangserythrozyten.

Mit dem erfindungsgemäßen Verfahren ist es demgemäß möglich, durch Elektroporation Erythrozyten mit Wirkstoffen zu beladen und ohne Hämoglobinverlust zu versiegeln und zu rejuvenilisieren, um auf diese Weise beladene Erythrozyten mit intaktem Stoffwechsel, normaler Größenverteilung und normaler Überlebenszeit zu schaffen.

Eine für die Durchführung der Elektroporation geeignete Vorrichtung weist mindestens einen Pulsgenerator und eine Porationskammer auf. Der Pulsgenerator liefert vorzugsweise elektrische Feldimpulse einer Feldstärke im Bereich von 1 bis 6 kV/cm mit einer Dauer im Bereich von 10 bis 100 Mikrosekunden. Die Porationskammer weist ein Volumen von vorzugsweise mindestens 100 ml auf und ist so konstruiert, daß alle Erythrozyten der gleichen Feldstärke ausgesetzt sind.

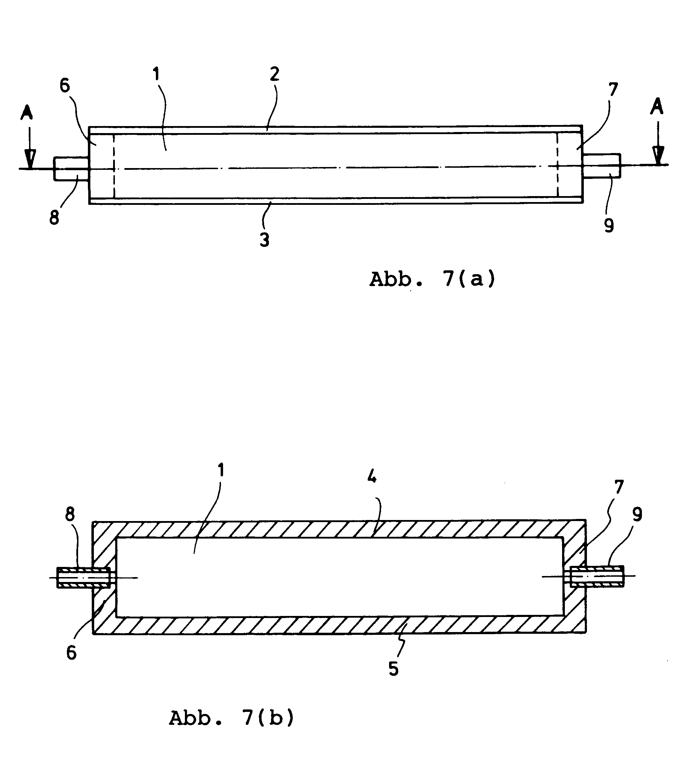

Eine erfindungsgemäß verwendbare Porationskammer ist in Abbildung 7 wiedergegeben. Teil (a) zeigt einen Längsschnitt durch die Porationskammer und Teil (b) einen Querschnitt durch die Achse A-A aus Teil (a). Ein Probenraum 1 wird von einer ersten Elektrode 2, einer zweiten Elektrode 3 und vier Seitenwänden 4, 5, 6, 7 gebildet. An der Seitenwand 6 ist ein Einlaß 8 und an der Seitenwand 7 ein Auslaß 9 für die Erythrozytensuspension angebracht. Die Seitenwände 4, 5, 6, 7 bestehen aus einem elektrisch isolierenden Material, vorzugsweise aus einem für die Erythrozyten nicht toxischen Kunststoff, der sterilisierbar ist. Die Elektroden 2 und 3 sind jeweils an einen Pol des Pulsgenerators angeschlossen. Da die Elektroden 2 und 3 parallel zueinander angeordnet sind und ihre Maße in Längs- und Querrichtung größer sind als ihr gegenseitiger Abstand, ist das zwischen ihnen bei Anliegen eines Impulses bestehende elektrische Feld bis auf Randeffekte weitgehend homogen. Alle Erythrozyten sind somit im wesentlichen der gleichen Feldstärke ausgesetzt.

Die Temperatur innerhalb der Porationskammer sollte etwa 0 bis 8 °C, vorzugsweise 0 bis 4 und insbesondere etwa 2 °C betragen.

Gemäß einer besonders bevorzugten Ausführungsform der Erfindung wird die Porationskammer automatisch mit der Erythrozytensuspension in dem erfindungsgemäßen Medium befüllt, wobei die Erythrozytenkonzentration etwa 50 bis 80 Vol.% betragen kann. Nach erfolgter Elektroporation wird die Porationskammer ebenfalls automatisch entleert und selbsttätig gereinigt bzw. sterilisiert.

Die Porationskammer kann ferner als Einweg-Behälter ausgestaltet sein. In diesem Fall können die Erythrozyten vor der Elektroporation in dem Einweg-Behälter durch Zentrifugieren von der physiologischen Kochsalzlösung abgetrennt, in dem erfindungsgemäßen Elektroporations-Medium suspendiert und mit dem EinwegBehälter in die Elektroporations-Vorrichtung eingesetzt werden.

Die Abtrennung der Erythrozyten von der physiologischen Kochsalzlösung nach dem Waschen kann auch nach anderen Verfahren wie beispielsweise durch Filtrieren erfolgen. In letzterem Falle kann das Filtrat anschließend mit dem Elektroporations-Medium durchspült und dieser Schicht automatisch in einem der Elektroporatons-Vorrichtungvorgeschalteten Geräteteil durchgeführt werden.

Die nachfolgenden Verfahrensstufen, d.h., das Beladen der Erythrozyten mit Wirkstoffen, das Versiegeln der Erythrozyten und das Rejuvenilisieren, werden vorteilhafterweise automatisch in Vorrichtungen durchgeführt, die der Elektroporations-Vorrichtung nachgeschaltet sind. Dazu müssen die den einzelnen Verfahrensstufen entsprechenden Geräteteile die jeweils geeignete Temperatur aufweisen und mit dem jeweils geeigneten Medium beschickt sein. Die Erythrozytensuspension kann taktweise oder intermittierend durch die Gesamtvorrichtung geleitet werden, so daß ein quasi-kontinuierlicher Betrieb möglich ist.

Das erfindungsgemäße Verfahren eignet sich grundsätzlich zur Beladung von Erythrozyten mit allen jenen Wirkstoffen, die per se nicht in der Lage sind, die intakte Zellmembran zu permeieren. Die gemäß einer besonders bevorzugten Ausführungsform der Erfindung mit allosterischen Hämoglobinaktivatoren und insbesondere mit Inositolhexaphosphat beladenen Erythrozyten geben Sauerstoff leichter und in größerer Menge ab, als dies bei normalen Erythrozyten der Fall ist. Dadurch kann mit den erfindungsgemäßen, IHP-beladenen Erythrozyten schon bei relativ niedrigen Hämatokritwerten eine normale Sauerstoffversorgung des Gewebes gewährleistet werden. Der niedrige Hämatokritwert ermöglicht wegen der daraus resultierenden geringen Viskosität des Blutes eine erfolgreiche Schocktherapie, da bei verbesserter Fließgeschwindigkeit des Blutes auch wieder mehr Sauerstoffträger in die verengten Kapillaren transportiert werden können.

Die erfindungsgemäß hergestellten Erythrozyten mit erhöhter Sauerstoffabgabebereitschaft sind ferner ein wertvolles therapeutisches Mittel für die Sauerstoffversorgung in Infarktgebieten sowie zur Behandlung von Tumorzellen mit ausschließlich anaerobem Stoffwechsel. Letztere werden zu einer schnellen Teilung angeregt und damit die Sensitivität gegenüber Cytostatika und Strahlen in einem übersäuerten Stofwechselmilieu gesteigert.

Die Erfindung wird nachfolgend anhand von Beispielen erläutert.

**Beispiel 1**

Bestimmung der Normalverteilung von Thrombozyten, Erythrozyten und Leukozyten.

Die normale Größenverteilung von Thrombozyten (plt), Erythrozyten (rbc) und Leukozyten (wbc) wurde durch Aufzeichnung der entsprechenden Streulichtsignale eines Laser-Hämatologiezählers (ELT 15 Ortho Instruments) in Histogramm-Form ermitttelt.

Zu diesem Zweck wurde eine von einem gesunden männlichen Erstspender stammende Blutprobe durch die Meßzelle des Geräts geführt, in der die Partikel im Flüssigkeitsstrom hydrodynamisch fokussiert werden. Damit können die Blutzellen einzeln vom Laserstrahl, der die Meßzelle durchstrahlt, erfaßt werden. Ein Teil des Laserlichts wird an den Partikeln gestreut, wobei die Intensität des Streulichtsignals von der Partikelgröße abhängig ist. Dieses Streulichtsignal wird von einem Detektor erfaßt, der in einem Winkel von 90° zum Laserstrahl angeordnet ist. Die Ergebnisse der Normal-Größenverteilung im Thrombozyten-, Erythrozyten- und Leukozytenkanal sind in Abbildung 1 wiedergegeben. Sie zeigen die gleichmäßige, einer Gauß'schen Kurve folgenden Größenverteilung gesunder Erythrozyten.

**Beispiel 2**

Herstellung von Leukozyten- und Thrombozyten-freier Erythrozytensuspensionen

Aus frisch entnommenen Blutkonserven von Dauerspendern wurden filtrierte Erythrozyten gewonnen. Zur Filtration wurde ein Erypur-g-2-Filter verwendet, welcher zu einer Leukozytenreduktion des Erythrozytenkonzentrats von 99,5% führte. Nach der Filtration konnten im ELT 15 praktisch keine Lymphozyten mehr nachgewiesen werden, während noch Thrombozyten vorhanden waren. Nach 3maligem Waschen in physiologischer NaCl-Lösung konnten auch keine Thrombozyten mehr nachgewiesen werden.

Die filtrierten und mit physiologischer NaCl-Lösung gewaschenen Erythrozyten wurden in physiologischer NaCl-Lösung suspendiert und die Größenverteilung wie in Beispiel 1 beschrieben ermittelt.

Die Ergebnisse sind in Abbildung 2 wiedergegeben. Sie zeigen, daß die Suspension keine Thrombozyten und im wesentlichen keine Leukozyten enthält, während die Erythrozyten nach wie vor der Normalverteilung entsprechen. Die in Abbildung 2 wiedergegebene Kurve diente als Standard für die Beurteilung der in den nachfolgenden Beispielen hergestellten Erythrozyten.

**Beispiel 3**

Beladung von Erythrozyten mit Raffinose als Modellsubstanz:

Das Erythrozytenkonzentrat gemäß Beispiel 2 wurde 3mal mit einem Medium gewaschen, welches je Liter 1,25 g NaCl, 1,8 g KCl, 1,14 g $NaH_2PO_4$ $\cdot$ $H_2O$, 0,51 g $MgCl_2$ $\cdot$ $6H_2O$, 2,8 g Natriumlactat und 55 g Glucose $\cdot$ $H_2O$ enthielt (Sterofundin$^R$ B). Die Temperatur des Mediums betrug etwa 4°C. Nach dem letzten Waschgang wurde der Überstand scharf abgezogen und anschließend der Hämatokrit des Erythrozytenkonzentrats auf einen Wert von 78 bis 80% HK eingestellt.

Jeweils 500 $\mu$l dieses Konzentrats wurde in 500 $\mu$l Sterofundin$^R$ B resuspendiert und bis zur Transfektion mindestens 15 Minuten lang im Eisbad auf + 2 ±1°C äquilibriert.

Die Suspension wurde anschließend in eine zwischen zwei Elektroden liegende Porationskammer mit einer Breite von 0,5 mm überführt und 80 $\mu$s lang einer Feldstärke von 3,6 kV/cm ausgesetzt.

Die Erythrozyten wurden anschließend in einer im Eisbad vorgekühlten Sterofundin$^R$ B-Lösung mit einem Gehalt an 5% Raffinose, bezogen auf die Gesamtlösung, aufgefangen. Die Mischung wurde 15 Minuten lang im Eisbad inkubiert und darauffolgend 10 Minuten lang in einer auf 4°C vorgekühlten Zentrifuge bei 500 g zentrifugiert.

Der Überstand wurde scharf abgezogen und verworfen.

Zum Versiegeln wurde das Erythrozytensediment dann in 500 $\mu$l einer Lösung der folgenden Zusammensetzung suspendiert:

0,1 Mol Kaliumchlorid

0,012 Mol Na-di-hydrogenphosphat

0,01 Mol L-Ascorbinsäure

0,003 Mol Magnesiumchlorid

30 Gew.% bezogen auf das Endvolumen der Lösung entsalzenes Humanalbumin.

Die Temperatur der Lösung betrug 37°C und der Ansatz wurde 30 Minuten lang bei 37°C im Wasserbad inkubiert.

Anschließend wurden die Erythrozyten über Nacht im Kühlschrank belassen und am nächsten Morgen die Hämolyserate durch Bestimmungt des freien Hämoglobins nach Simmons (Technical Hematology, 3. Auflage, Seite 12, (1980)) gemessen. Es konnte kein freies Hämoglobin nachgewiesen werden.

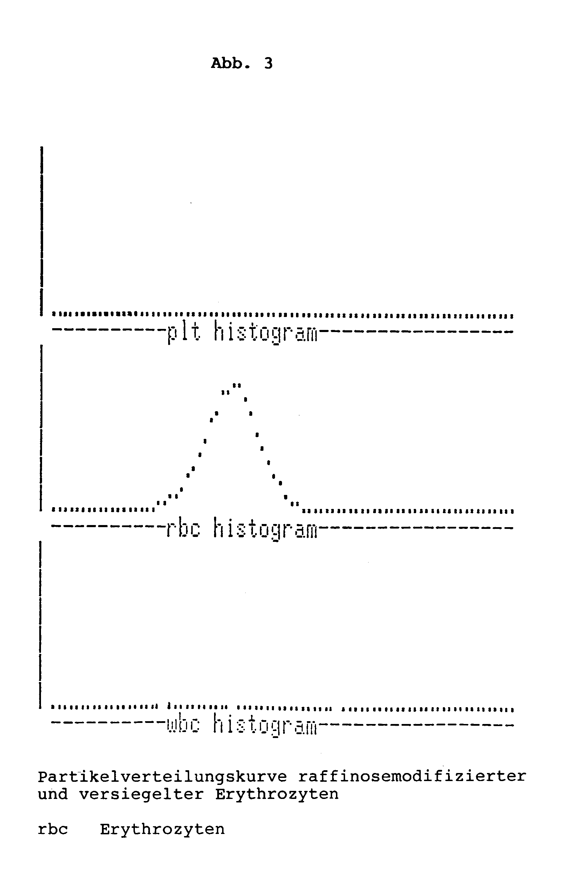

Die Partikelverteilungskurve der versiegelten Erythrozyten ist in Abbildung 3 dargestellt. Sie zeigt, daß die Größenverteilung der nach dem erfindungsgemäßen Verfahren beladenen und versiegelten Erythrozyten mit der Normalverteilungskurve völlig identisch ist.

**Beispiel 4**

Nachweis der Beladung mit Raffinose

Die versiegelten Zellen gemäß Beispiel 3 wurden 30 Minuten lang bei 57° C im Wasserbad hämolysiert und anschließend das Eiweiß nach dem Verfahren von Carrez ("Methoden der biochemischen Analytik und Lebensmittelanalytik mit Fest-Combinationen", Biochemische Analytik, Lebensmittelanalytik, Boehringer Mannheim, Seite 99) ausgefällt.

Nach Zugeben von 100 $\mu$l 0,5 Natronlauge wurde das Gemisch auf Zimmertemperatur abgekühlt und der Niederschlag in eine Eppendorf-Mikroliter-Zentrifuge abzentrifugiert. Der Überstand wurde zur enzymatischen Bestimmung der Raffinose mit $\alpha$-Galactosidase (Raffinose UV-Test Boehringer Mannheim) verwendet. Als Leerwert wurde der vom letzten Waschgang der versiegelten Erythrozyten vor Hämolyse erhaltene Überstand eingesetzt. Die Extinktionsdifferenz zwischen Leerwert und Meßwert ist proportional der Raffinosekonzentration.

Die Ablesung erfolgte bei 334 nm, die Extinktion des Leerwertes betrug 0,003 und diejenige der Probe 0,340.

Das Ergebnis zeigt, daß die versiegelten Erythrozyten gemäß Beispiel 3 mit Raffinose beladen waren.

**Beispiel 5**

Beladung von Erythrozyten mit Inositolhexaphosphat

Zur Herstellung einer natriumarmen Inositolhexaphosphat-Lösung wurden 5 g des handelsüblichen stark basischen Natriumsalzes ($Na_{12}IHP$) in 100 ml Aqua dest. gelöst. Der pH-Wert dieser Lösung betrug 10,6. Unter ständigem Rühren wurde solange saures Ionenaustauscherharz vom Typ Dowex$^R$ 50 W-X 16 (Biorat) hinzugefügt, bis ein pH-Wert von 1,4 erreicht war.

Der Ionenaustauscher wurde anschließend über eine Filterfritte entfernt und das klare Filtrat durch Zugeben von ca. 800 $\mu$l 10 M KOH auf einen pH-Wert von 7,4 eingestellt. Das erhaltene Kalium-Natriumsalz der Inositolphoshorsäure enthielt folgende Elektrolytkonzentrationen:

| | |
|---|---|
| $Na^+$ | 30,5 mVal |
| $K^+$ | 144,28 mVal. |

Die Beladung von Erythrozyten mit Inositolhexaphosphat erfolgte nach dem in Beispiel 3 beschriebenen Verfahren mit dem Unterschied, daß anstelle der Raffinose die oben beschriebene Inositolhexaphosphatlösung bis zu einer IHP-Konzentration von etwa 5% bezogen auf die Gesamtlösung zugegeben wurde.

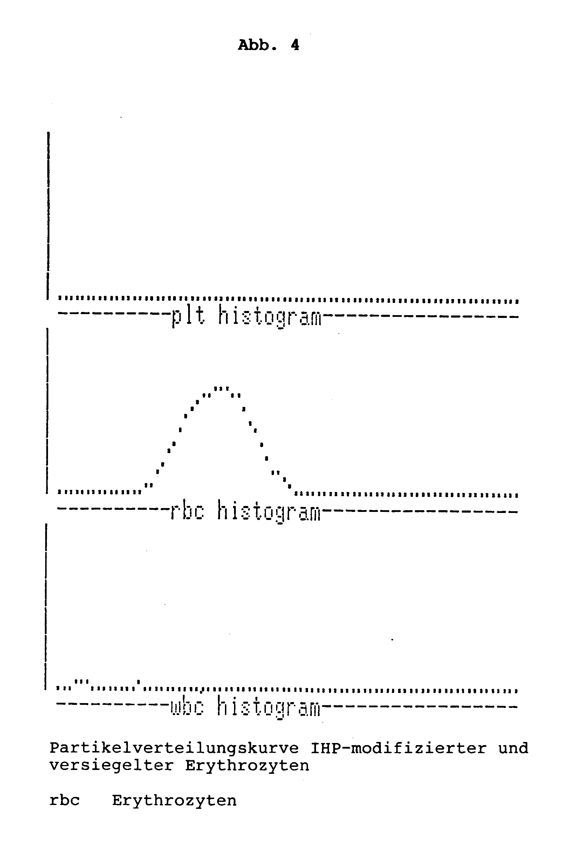

Die Größenverteilung der nach den Verfahren gemäß Beispiel 3 erhaltenen, mit Inositolhexaphosphat beladenen und versiegelten Erythrozyten ist in Abbildung 4 wiedergegeben. Sie zeigt, daß auch die Beladung mit dieser bevorzugten Substanz nach dem erfindungsgemäßen Verfahren zu Erythrozyten mit normaler Größenverteilung führt.

**Beispiel 6**

Sauerstoffbindungskurve Inositolhexaphosphat-modifizierter Erythrozyten

Erythrozyten hergestellt gemäß den Beispielen 3 und 5 wurden jeweils wie in Beispiel 3 beschrieben versiegelt und 10 Minuten lang bei 500 g zentrifugiert. Der Überstand wurde scharf abgezogen. Anschließend wurde 3mal mit 8 ml eiskalter Stabilisatorlösung (handelsübliche CPD-A-Stabilisatorlösung für Erythrozytenkonzentrate) gewaschen, um freies Inositolhexaphosphat aus dem Suspensionsmedium zu entfernen. Nach dem letzten Waschgang wurde die Stabilisatorlösung scharf abgezogen, verworfen und durch 500 $\mu$l AB-Plasma ersetzt.

Die Sauerstoffbindungskurven normaler Erythrozyten sowie der nach den Beispielen 4 und 5 hergestellten Raffinose- bzw. IHP-beladenen Erythrozyten wurden nach Standardverfahren ( vgl. Opitz,E. und H.

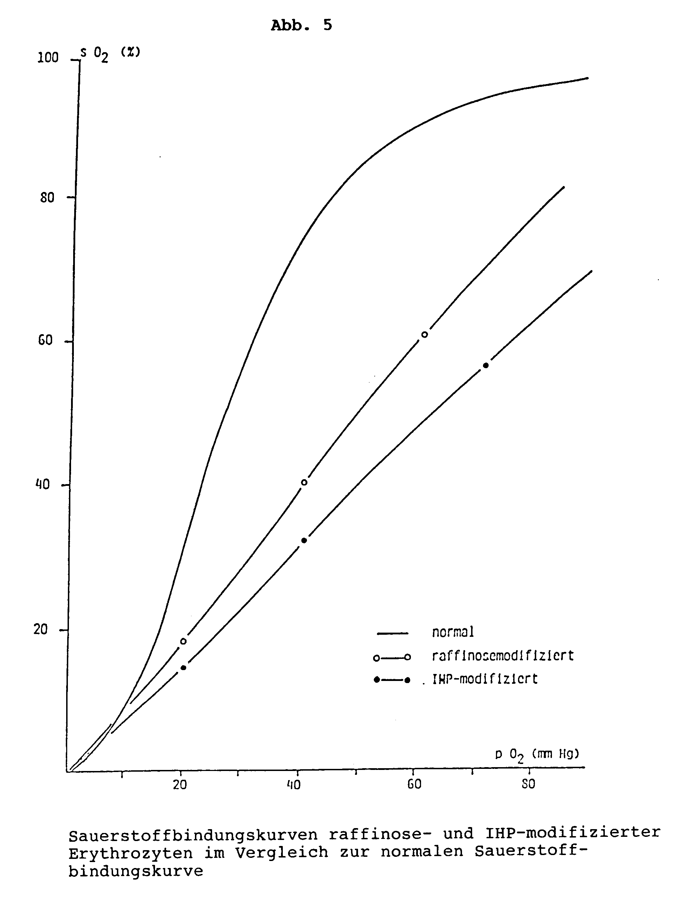

Bartels: Gasanalyse, in Hoppe-Seyler, Thierfelder, Handbuch der physiologischen und pathologisch-chemischen Analyse, 10. Auflagen, Bd.II) erstellt. Die Ergebnisse sind in Abbildung 5 wiedergegeben.

Sie zeigen, daß im Vergleich zur normalen Sauerstoffbindungskurve bereits die Bindungskurve der Raffinose-modifizierten Erythrozyten eine Rechtsverschiebung aufweist. Diese war aufgrund der hohen $H^+$-Ionenkonzentration zu erwarten. Demgegenüber zeigt die $O_2$-Bindungskurve der IHP-modifizierten Erythrozyten eine deutlich stärkere Rechtsverschiebung, die nur auf das eingeschleuste Inositolhexaphosphat zurückgeführt werden kann.

**Beispiel 7**

Rejuvenilisieren der Erythrozyten nach Versiegelung

500 $\mu$l einer über Nacht im Kühlschrank belassenen Erythrozytensuspension gemäß Beispiel 3 wurden mit 150 $\mu$l einer wässrigen Rejuvenilisierungslösung der folgenden Zusammensetzung versetzt:

| | |
|---|---|
| Glucose | 3,44 g/l |
| Inosin | 26,8 g/l |
| Na-Pyruvat | 13,75 g/l |
| Adenin | 0,675 g/l |
| Di-Na-hydrogenphosphat 7$H_2$O | 55,0 g/l |
| Benzylalkohol | 10,0 g/l |

und etwa 12 Stunden lang bei 4°C inkubiert.

Der Erfolg der Rejuvenilisierung wurde anschließend durch den Nachweis einer ATP-Neusynthese überprüft.

Die Bestimmung wurde nach H. Adams, "Adenosine 5'-Triphosphate Determination with Phosphoglycerate Kinase", Methods of Enzymatic Analysis, Bergmeyer, H.U., Akademic, Press., New York, Seiten 539 bis 543 (1963) durchgeführt.

Vor der Messung wurde die jeweilige Erythrozytensuspension dreimal in eiskalter physiologischer Kochsalzlösng gewaschen und anschließend eine Eiweißfällung in 12%-iger Trichloressigsäure durchgeführt, um das Hämoglobin und andere Eiweißbestandteile des Erythrozyten aus dem Reaktionsansatz zu entfernen.

Die ATP-Bestimmung wurde an dem klaren Überstand durchgeführt, wobei die ATP-Konzentration der Erythrozyten in der Ausgangssuspension, der Suspension direkt nach der Versiegelung und ferner nach Abschluß der Rejuvenilisierung bestimmt wurde.

Die Meßergebnisse sind in der nachfolgenden Tabelle wiedergegeben:

| Nachweis der ATP-Neusynthese von rejuvenilisierten Erythrozyten | |
|---|---|
| Material | ATP-Konzentration |
| unbehandelte Erythrozyten | 4,21 $\mu$Mol/g Hb |
| versiegelte Erythrozyten | 1,05 $\mu$Mol/g Hb |
| versiegelte und rejuveniliserte Erythrozyten | 4,03 $\mu$Mol/g Hb |

Die Ergebnisse zeigen, daß der ATP-Gehalt der Erythrozyten sowohl in der Ausgangssuspension als auch in den Suspensionen der versiegelten Erythrozyten nach Rejuvenilisierung im Rahmen der Methoden abhängigen Schwankungsrate identisch ist. Bezogen auf den Hämoglobingehalt wurde eine ATP-Neusynthese von fast 3 $\mu$Mol ATP/g Hämoglobin gefunden. Der Stoffwechsel der rejuvenilisierten Erythrozyten war demgemäß in Takt.

**Vergleichsbeispiel**

Beladung von Erythrozyten durch hypotone Lyse in Dialysemembranen nach Ropars

Zur hypotonen Lyse in Dialysemembranen wurden 10 ml des Erythrozytenkonzentrats gemäß Beispiel 2 auf 4°C abgekühlt und auf einen Hematokritwert von 70% eingestellt. Anschließend wurden die Erythrozyten in einen zuvor mit dest. Wasser gewässerten Visking Dialyseschlauch (20/32) mit einer Ausschlußgrenze von 10 000 bis 20 000 Dalton eingefüllt.

Der gefüllte Schlauch wurde anschließend 90 Minuten lang bei 4°C gegen 150 ml hypotonen $K^+$-Phosphat-Puffer der (5 mM, pH 7,4) dialysiert. Zur Vermeidung einer Sedimentation der Erythrozyten wurde der Schlauch mit 6 UpM rotierend bewegt. Nach Ablauf der Lysezeit wurde der Dialyseschlauch zur Versiegelung der Erythrozyten in isotone Pufferlösung einer Temperatur von 37°C der folgenden Zusammensetzung überführt:

| NaCl | 137,00 mMol |
| KCl | 2,6 mMol |
| $Na_2HPO_4$ | 7,37 mMol |
| $KH_2PO_4$ | 1,47 mMol. |

Der pH-Wert der Pufferlösung betrug 7,4.

Zur Versiegelung der Zellen wurde der Schlauch 30 Minuten lang in der oben genannten Pufferlösung belassen und wiederum rotierend bewegt.

Nach der Versiegelung wurden die Zellen in physiologischer NaCl-Lösung gewaschen und vorsichtig zentrifugiert (500 g), um mechanische Hämolysen zu vermeiden. Die Größenverteilung der Partikel wurde anschließend wie zuvor beschrieben gemessen. Die Partikelverteilungskurve ist in Abbildung 6 dargestellt. Sie zeigt, daß die Größenverteilung der nach dem Ropars-Verfahren beladenen und versiegelten Erythrozyten wesentlich von der Normalverteilungskurve abweicht.

**Patentansprüche**

1. Verfahren zum Herstellen vitaler Wirkstoff-beladener Humanerythrozyten durch Elektroporation der Erythrozytenmembranen, Beladen der porösen Erythrozyten mit dem vorgesehenen Wirkstoff, Versiegeln der Membranen und Rejuvenilisieren der Erythrozyten, **dadurch gekennzeichnet**, daß man

   a) die Elektroporation bei 0 bis 8°C in einem Medium durchführt, dessen Leitfähigkeit 5 bis 8 mS beträgt und welches eine Osmolarität von 290 bis 500 Osmol/l aufweist, wobei das Verhältnis von $Na^+$:K+-Ionen in dem Medium im Bereich von 3:1 bis 1:3 liegt,

   b) die in Stufe a) gewonnenen Erythrozyten bei 0 bis 8°C mit einer Lösung des vorgesehenen Wirkstoffs inkubiert,

   c) die in Stufe b) gewonnenen Erythrozyten versiegelt, indem man sie bei 36°C bis 41°C in einem Medium inkubiert, das 80 bis 100 mVal/l $K^+$-Ionen, 0 bis 20 mVal $Na^+$-Ionen und 25 bis 35 Gew./Vol.% Humanalbumin oder Humanalbumin-Ersatzstoff enthält, und

   d) die in Stufe c) gewonnenen Erythrozyten nach bekannten Verfahren rejuvenilisiert.

2. Verfahren nach den Ansprüchen 1 oder 2, **dadurch gekennzeichnet**, daß man die Elektroporation in Stufe a) bei einer Feldstärke von 1 bis 6 kV/cm, vorzugsweise 3 bis 4 kV/cm, einer Pulsdauer von 20 bis 100 μs, vorzugsweise 80 μs und bei einer Temperatur von 0 bis 4°C, vorzugsweise 2°C durchführt.

3. Verfahren nach den Ansprüchen 1 oder 2, **dadurch gekennzeichnet**, daß man in Stufe a) ein Medium verwendet, welches etwa 50 g/l Glucose, etwa 54 mMol/l $Na^+$, etwa 24 mMol/l $K^+$, etwa 2,5 mMol/l $Mg^{++}$, etwa 51 mMol/l $Cl^-$, etwa 25 mMol/l Lactat und etwa 7,5 mMol/l $H_2PO^{-4}$ enthält.

4. Verfahren nach den Ansprüchen 1 oder 2, **dadurch gekennzeichnet**, daß man in Stufe b) als Wirkstoff einen allosterischen Hämoglobinaktivator verwendet.

**5.** Verfahren nach Anspruch 3, **dadurch gekennzeichnet**, daß man als Wirkstoff Inositolhexaphosphat verwendet.

**6.** Verfahren nach den Ansprüchen 1 bis 5, **dadurch gekennzeichnet**, daß man in Stufe c) ein Medium verwendet, welches zusätzlich 2 bis 5 mVal/l $Mg^{2+}$-Ionen sowie gegebenenfalls 5 bis 20 mMol/l L-Ascorbinsäure enthält.

**7.** Verfahren nach den Ansprüchen 1 bis 6, **dadurch gekennzeichnet**, daß die Anionen in dem in Stufe c) verwendeten Medium durch 1,5 bis 2,5 mMol anorganische Phosphationen und der verbleibende Teil gegebenenfalls durch Chlorionen gebildet werden.

**8.** Vorrichtung zur Durchführung des Verfahrens nach den Ansprüchen 1 bis 7 mit einem Pulsgenerator und einer Porationskammer, **dadurch gekennzeichnet**, daß der Pulsgenerator elektrische Feldimpulse einer Feldstärke im Bereich von 1 bis 6 kV/cm und einer Dauer von 10 bis 100 Mikrosekunden erzeugt.

**9.** Vorrichtung nach Anspruch 8, **dadurch gekennzeichnet**, daß die Porationskammer ein Volumen von mindestens 100 ml aufweist.

**10.** Vorrichtung nach den Ansprüchen 8 oder 9, **dadurch gekennzeichnet**, daß das Befüllen, Entleeren oder Reinigen der Porationskammer automatisch erfolgt.

**11.** Vorrichtung nach den Ansprüchen 8 oder 9, **dadurch gekennzeichnet**, daß die Porationskammer eine Einweg-Kammer ist.

**12.** Vorrichtung nach einem der Ansprüche 8 bis 10, **dadurch gekennzeichnet**, daß das Befüllen und Entleeren der Porationskammer kontinuierlich oder quasi-kontinuierlich erfolgt.

**13.** Vorrichtung nach Anspruch 12, **dadurch gekennzeichnet**, daß der Porationskammer Kammern zur kontinuierlichen oder quasikontinuierlichen Durchführung der Verfahrensstufen b) bis d) gemäß den Ansprüchen 1 bis 7 nachgeschaltet sind.

Abb. 1

----------plt histogram----------------

----------rbc histogram----------------

----------wbc histogram----------------

Normalverteilung von Thrombozyten, Erythrozyten und
Leukozyten im Histogrammausdruck des ELT 15

plt   Thrombozyten
rbc   Erythrozyten
wbc   Leukozyten

Abb. 2

----------plt histogram----------------

----------rbc histogram----------------

----------wbc histogram----------------

Histogrammausdruck einer mit dem ELT 15 gemessenen
filtrierten und mit physiologischer NaCl-Lösung
gewaschenen Erythrozytensuspension

rbc    Erythrozyten

**Abb. 3**

```
|
|
|
|
|
|
| ...................................................................
|----------plt histogram----------------------
|                      ""
|                     . ".
|                    .    .
|                   .      .
|                  .        .
|                 .          .
|                .            .
| ............'..'            ".
|..............'                ".......................
|----------rbc histogram----------------------
|
|
|
|
|
|
| ................. ......... ............... ..........................
|----------wbc histogram----------------------
```

Partikelverteilungskurve raffinosemodifizierter
und versiegelter Erythrozyten

rbc    Erythrozyten

**Abb. 4**

Partikelverteilungskurve IHP-modifizierter und versiegelter Erythrozyten

rbc    Erythrozyten

## Abb. 5

Sauerstoffbindungskurven raffinose- und IHP-modifizierter Erythrozyten im Vergleich zur normalen Sauerstoff- bindungskurve

**Abb. 6**

Partikelverteilungskurve nach hypotoner Lyse
in Dialysemembranen

rbc     Erythrozyten
wbc     Leukozyten

Abb. 7(a)

Abb. 7(b)

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| E | DE-A-3 925 680 (KRÜSS GmbH)(07-02-191)<br>* Insgesamt *<br>– – – | 1-13 | A 61 K 9/50<br>A 61 K 35/18 |
| T | FEBS, Band 275, Nr. 1,2, November 1990, Seiten 117-120; Y. MOUNEIMNE et al.: "Stable rightward shifts of the oxyhemoglobin dissocation curve induced by encapsulation of inositol hexaphosphate in red blood cells using electroporation"<br>* Insgesamt *<br>– – – | 1-13 | |
| A | EP-A-0 362 758 (TEXAS A & M UNIVERS. SYSTEM TEXAS A M UNIVERSITY)<br>* Spalte 3, Zeile 30 - Spalte 6, Zeile 17; Spalten 7,8, Beispiel 1 *<br>– – – | 1 | |
| A | NUCLEIC ACIDS RESEARCH, Band 15, Nr. 3, Februar 1987, Seiten 1311-1326; G. CHU et al.: "Electroporation for the efficient transfection of mammalian cells with DNA"<br>* Seiten 1314-1319 *<br>– – – | 1 | |
| D,A | EP-A-0 101 341 (CNRS et al.)<br>– – – – – | | |

**RECHERCHIERTE SACHGEBIETE (Int. Cl.5)**

A 61 K
C 12 N

Der vorliegende Recherchenbericht wurde für alle Patentansprüche erstellt

| Recherchenort | Abschlußdatum der Recherche | Prüfer |
|---|---|---|
| Den Haag | 15 Mai 91 | BENZ K.F. |